(19) Europäisches Patentamt
European Patent Office
Office européen des brevets

(11) **EP 2 334 699 B1**

(12) **EUROPEAN PATENT SPECIFICATION**

(45) Date of publication and mention
of the grant of the patent:
**11.09.2013 Bulletin 2013/37**

(21) Application number: **09815645.8**

(22) Date of filing: **21.09.2009**

(51) Int Cl.:
**C07K 14/505** (2006.01)

(86) International application number:
**PCT/EP2009/006783**

(87) International publication number:
**WO 2010/034442 (01.04.2010 Gazette 2010/13)**

(54) **PURIFICATION OF ERYTHROPOIETIN**

**AUFREINIGUNG VON ERYTHROPOIETIN**

**PURIFICATION DE L'ÉRYTHROPOÏÉTINE**

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **23.09.2008 EP 08016678**

(43) Date of publication of application:
**22.06.2011 Bulletin 2011/25**

(73) Proprietor: **F. Hoffmann-La Roche AG
4070 Basel (CH)**

(72) Inventors:
• **SCHMALZ, Christian
82362 Weilheim (DE)**
• **SCHMELZER, Antje
12439 Berlin (DE)**

(74) Representative: **Skolaut, Alexander
Roche Diagnostics GmbH
Patentabteilung TR-E
Nonnenwald 2
D-82377 Penzberg (DE)**

(56) References cited:
EP-A- 1 428 878          WO-A-99/28346
WO-A-03/080852          WO-A-2005/121173
US-A1- 2004 147 431

EP 2 334 699 B1

SEQUENCE LISTING

<110> F. Hoffmann-La Roche AG

<120> Reinigung von Erythropoietin

<130> 25389 WO

<150> EP08016678.8
<151> 2008-09-23

<160> 2

<170> PatentIn version 3.2

<210> 1
<211> 165
<212> PRT

<213> Homo sapiens

<400> 1

```
        Ala Pro Pro Arg Leu Ile Cys Asp Ser Arg Val Leu Glu Arg Tyr Leu
        1               5                   10                  15

        Leu Glu Ala Lys Glu Ala Glu Asn Ile Thr Thr Gly Cys Ala Glu His
                    20                  25                  30

        Cys Ser Leu Asn Glu Asn Ile Thr Val Pro Asp Thr Lys Val Asn Phe
                    35                  40                  45

        Tyr Ala Trp Lys Arg Met Glu Val Gly Gln Gln Ala Val Glu Val Trp
            50                  55                  60

        Gln Gly Leu Ala Leu Leu Ser Glu Ala Val Leu Arg Gly Gln Ala Leu
        65                  70                  75                  80

        Leu Val Asn Ser Ser Gln Pro Trp Glu Pro Leu Gln Leu His Val Asp
                        85                  90                  95

        Lys Ala Val Ser Gly Leu Arg Ser Leu Thr Thr Leu Leu Arg Ala Leu
                        100                 105                 110

        Gly Ala Gln Lys Glu Ala Ile Ser Pro Pro Asp Ala Ala Ser Ala Ala
                    115                 120                 125

        Pro Leu Arg Thr Ile Thr Ala Asp Thr Phe Arg Lys Leu Phe Arg Val
            130                 135                 140

        Tyr Ser Asn Phe Leu Arg Gly Lys Leu Lys Leu Tyr Thr Gly Glu Ala
            145                 150                 155                 160

        Cys Arg Thr Gly Asp
                    165
```

<210> 2
<211> 166
<212> PRT
<213> Homo sapiens

<400> 2

```
Ala Pro Pro Arg Leu Ile Cys Asp Ser Arg Val Leu Glu Arg Tyr Leu
1               5               10              15

Leu Glu Ala Lys Glu Ala Glu Asn Ile Thr Thr Gly Cys Ala Glu His
            20              25              30

Cys Ser Leu Asn Glu Asn Ile Thr Val Pro Asp Thr Lys Val Asn Phe
        35              40              45

Tyr Ala Trp Lys Arg Met Glu Val Gly Gln Gln Ala Val Glu Val Trp
    50              55              60

Gln Gly Leu Ala Leu Leu Ser Glu Ala Val Leu Arg Gly Gln Ala Leu
65              70              75              80

Leu Val Asn Ser Ser Gln Pro Trp Glu Pro Leu Gln Leu His Val Asp
            85              90              95

Lys Ala Val Ser Gly Leu Arg Ser Leu Thr Thr Leu Leu Arg Ala Leu
            100             105             110

Gly Ala Gln Lys Glu Ala Ile Ser Pro Pro Asp Ala Ala Ser Ala Ala
        115             120             125

Pro Leu Arg Thr Ile Thr Ala Asp Thr Phe Arg Lys Leu Phe Arg Val
    130             135             140

Tyr Ser Asn Phe Leu Arg Gly Lys Leu Lys Leu Tyr Thr Gly Glu Ala
145             150             155             160

Cys Arg Thr Gly Asp Arg
            165
```

## Description

Herein is reported a method for purifying erythropoietin (EPO) using a hydroxyapatite chromatography. In addition a method is described for depleting host cell proteins as well as a method for modifying the isoform distribution of an EPO composition.

## Background of the Invention

Erythropoietin (EPO) is a human glycoprotein which stimulates the production of red blood cells. Its action and therapeutic application are for example described in detail in EP 0 148 605, EP 0 205 564, EP 0 209 539 and EP 0 411 678, as well as in Huang, S.L., Proc. Natl. Acad. Sci. USA (1984) 2708-2712, Lai, P.H., et al., J. Biol. Chem. 261 (1986) 3116-3121, and Sasaki, H., et al., J. Biol. Chem. 262 (1987) 12059-12076.

EPO occurs only in very low concentrations in the blood plasma of healthy persons. The isolation of human EPO from the urine of patients with aplastic anemia is known (Miyake, T., et al., J. Biol. Chem. 252 (1977) 5558-5564). A seven step method is described which comprises ion exchange chromatography, ethanol precipitation, gel filtration and adsorption chromatography. EP 0 148 605 and EP 0 205 564 report the production of recombinant human EPO in CHO cells. A method for purifying recombinantly produced EPO was described by Nobuo, I., et al., J. Biochem. 107 (1990) 352-359.

Further methods for producing and purifying erythropoietin are known and described inter alia in EP 0 148 605, EP 0 205 564, EP 0 255 231, EP 0 830 376, EP 0 267 678, EP 0 228 452, EP 1 127 063, EP 0 209 539, EP 0 358 463, EP 1 010 758, EP 0 820 468, EP 0 984 062, EP 0 640 619, EP 0 428 267, EP 1 037 921 and in Lai, P.H., et al., J. Biol. Chem. 261 (1986) 3116-3121, Broudy, V.C., et al., Arch. Biochem. Biophys. 265 (1988) 329-336, Zou, Z., et al., Journal of Chrom. 16 (1998) 263-264, Inoue, N., et al., Biol. Pharm. Bull. 17 (1994) 180-184, Qian, R.L., et al., Blood 68(1) (1986) 258-262, Krystal, G., et al., Blood 67(1) (1986) 71-79, Lange, R.D., et al., Blood Cells 10(2-3) (1984) 305-314, Sasaki, R., et al., Methods Enzymol. 147 (1987) 328-340, Ben Ghanem, A., et al., Prep. Biochem. 24 (1994) 127-142, Cifuentes, A., et al., J. Chrom. A 830 (1999) 453-463; and Gokana, A., et al., J. Chromat. A 791 (1997) 109-118.

EP 1 394 179 reports a method for producing erythropoietin free from animal proteins. The production of erythropoietin by endogenous gene activation with viral promoters is the subject matter of EP 0 986 644. A method for producing recombinant glycoproteins and in particular erythropoietin is reported in EP 1 428 878. A method for producing a desired profile of erythropoietin glyco-isoforms is reported in EP 1 492 878.

EP 1 428 878 reports a process for the production and purification of erythropoietin. A method for purifying erythropoietin is reported in WO 2005/121173. In WO 99/28346 an erythropoietin with high specific activity is reported. An erythropoietin composition is reported in US 2004/147431. In WO 03/080852 a process for the preparation of a desired erythropoietin glyco-isoform profile is reported.

## Summary of the Invention

The production and purification of recombinant erythropoietin requires several steps, in particular chromatography steps.

A first aspect of the invention is a method for producing erythropoietin comprising at least one chromatography step with a stationary phase containing hydroxyapatite comprising the steps of

i) bringing an erythropoietin containing solution containing Calcium-ions at a concentration of 5 mM comprising 20 mM TRIS-HCl, pH 6.9 ± 0.2, into contact with a stationary phase containing ceramic hydroxyapatite which has been equilibrated with a solution containing Calcium-ions at the same concentration of 5 mM comprising 20 mM TRIS-HCl, 0.25 M NaCl, 9% (v/v) 2-propanol, pH 6.9 ± 0.2,

ii) passing a solution which contains Calcium-ions at a concentration of less than 0.1 mM over the stationary phase containing ceramic hydroxyapatite comprising 20 mM TRIS-HCl, 0.25 M NaCl, 9% (v/v) 2-propanol, pH 6.9 ± 0.2, and

iii) passing a solution which contains Calcium-ions at a concentration of less than 0.1 mM comprising 10 mM TRIS-HCl, pH 6.9 ± 0.2, over the stationary phase containing ceramic hydroxyapatite and thereby producing erythropoietin.

In one embodiment the produced erythropoietin is depleted of de-O-EPO species and de-N-EPO species compared to the erythropoietin containing solution in i). In a further embodiment the method comprises at least one additional chromatography step in addition to the chromatography step with a stationary phase containing hydroxyapatite. In a further embodiment all chromatography steps are each based on different chromatographic principles, whereby each chromatographic principle is used only once in the method. In a further embodiment the chromatographic principle(s) is/are selected from affinity chromatography, hydrophobic interaction chromatography, reversed phase chromatography, gel permeation chromatography, cation exchange chromatography, and/or anion exchange chromatography. In a further embodiment the sequence of chromatography steps are affinity chromatography, hydrophobic interaction chromatography, chromatography on a stationary phase containing hydroxyapatite, optionally reversed phase chromatography, and anion exchange chromatography. In one embodiment the method according to the invention comprises optional intermediate steps such as salt precipitation, concentration, diafiltration, ultrafiltration, dialysis, and/or ethanol precipitation in addition to the chromatography steps.

In another embodiment the solution used in the chromatography in step ii) and/or iii) contains 5 mM sodium - or potassium -phosphate.

Yet a further aspect of the present invention is a method for producing recombinant erythropoietin comprising cultivating host cells, which contain an erythropoietin-encoding nucleic acid, in a suitable medium under suitable conditions to produce erythropoietin, and isolating the erythropoietin from the culture medium or the cells. The cells are cultured in suspension in one embodiment. In a further embodiment the cells are cultured in a fermenter, and in particular in a fermenter with a volume of 10 l to 50,000 l.

The isolation of erythropoietin from the culture medium comprises the following steps:

(a) applying the cell culture supernatant to an affinity chromatography material and recovering/collecting the fractions containing erythropoietin,
(b) optionally applying the fractions of (a) to a hydrophobic interaction chromatography material and recovering/collecting the fractions containing erythropoietin,
(c) applying the fractions of (a) or (b) to a stationary phase containing hydroxyapatite and recovering/collecting the fractions containing erythropoietin using a method according to the present invention, and
(d) concentrating or/and passing the fractions of (c) over a reversed phase HPLC material.

Step (a) of the purification method comprises passing the cell supernatant, which can optionally be pretreated, over an affinity chromatography material. In one embodiment the affinity chromatography material is one to which a blue dye has been coupled. An example is Blue Sepharose. In one embodiment, after elution from the affinity chromatography material, the eluate containing erythropoietin is passed over a hydrophobic interaction chromatography material if a culture medium with a serum content of $\geq$ 2% (v/v) was used for the fermentation/its production. In one embodiment the hydrophobic interaction chromatography material is butyl Sepharose. The eluate from step (a) or, if used, step (b) is passed over a stationary phase containing hydroxyapatite in step (c) and the eluate containing erythropoietin is isolated. In one embodiment the concentrating is by means of exclusion chromatography e.g. membrane filtration. In this case the use of e.g. a membrane with an exclusion size of 10 kDa has proven to be advantageous. The erythropoietin that can be obtained with the method according to the invention can contain $\alpha$- 2, 3- linked or/and $\alpha$- 2, 6- linked sialic acid residues.

Another aspect of the invention is a method for producing poly(ethyleneglycol) conjugated erythropoietin comprising the following steps:

a) covalently linking, i.e. PEGylating, erythropoietin using an activated poly(ethyleneglycol) reagent,

b) purifying the poly(ethyleneglycol) conjugated erythropoietin by two successive cation exchange chromatography steps and thereby producing poly(ethyleneglycol) conjugated erythropoietin,

in which the erythropoietin employed in step a) is purified or produced by a method according to the present invention.

## Detailed Description of the Invention

Whether different substances can be chromatographically separated from each another, depends above all on the conditions under which the chromatography is carried out, in addition on a good column packing and on the choice of the chromatography material (stationary phase). This includes the way in which impurities and the product are eluted in addition to the choice of the buffer system.

The present invention reports a method for purifying erythropoietin comprising at least one chromatography step with a stationary phase containing hydroxyapatite comprising the following steps:

i) bringing an erythropoietin solution to be purified containing Calcium-ions at a first concentration into contact with a stationary phase containing hydroxyapatite over which a volume of a solution containing Calcium-ions at a second concentration has been passed,
ii) passing a volume of a solution containing a lower concentration of Calcium-ions than the solutions from i), i.e. at a third concentration, over the stationary phase containing hydroxyapatite, whereby during this passing the erythropoietin remains bound to/is not detached from the stationary phase containing hydroxyapatite, and

iii) passing a volume of a solution containing less than 0.5 mM Calcium-ions, i.e. at a fourth concentration, over the stationary phase containing hydroxyapatite from ii), whereby the erythropoietin detaches and elutes from the stationary phase containing hydroxyapatite.

In one embodiment the first and second and/or third and fourth concentration are different. In another embodiment the third and fourth Calcium-ion concentration are 10% or less of the first and second Calcium-ion concentration.

Erythropoietin, which is also referred to as EPO in the following, is understood as a protein which has the biological ability to stimulate differentiation and division processes in erythroid precursor cells and, thus, to provide erythrocytes. This protein is preferably human erythropoietin and consists of 165 or 166 amino acids (SEQ ID NO: 1 and 2) with a molecular weight of about 34- 38 kDa. The glycosyl residues account for about 40% of the molecular weight. Derivatives and fragments of EPO such as a PEGylated EPO, i.e. a poly (ethyleneglycol) conjugated erythropoietin, which have analogous biological properties and comprise erythropoietin obtained by culturing an EPO- producing host cell, can also be prepared by the method according to the invention. The DNA sequence and protein sequence of human EPO is described among others in EP 0 205 564 and EP 0 209 539.

The structure of EPO comprises two disulfide bridges and several sugar chains. The sugar chains, which are also referred to as glycosyl residues in the following, are bound to the protein backbone. Some chains are bound N-glycosidically to the protein via asparagine residues and a chain is bound O-glycosidically to the protein via a serine residue.

Sialic acid (N- acetylneuraminic acid) is usually incorporated at the end of a (branched or unbranched) glycosyl residue. The glycosyl residue can subsequently no longer be extended. Sialic acid can only be linked to a $\alpha 2$- 3 bond of Gal ($\beta$1- 4) GlcNAc (Nimtz, M., et al., Eur. J. Biochem. 213 (1993) 39- 56) .

The term "antennarity" or "antennarity of a glycosyl residue" refers to the degree of branching of the glycosyl residue. If a glycosyl residue for example has two arms, the glycosyl residue is biantennary i.e. it is a biantennary glycosyl residue. The glycosyl residues can have repetitive sections, so- called repeats. These are regions in which the sequence GlcNAc and galactose (N- acetyllactisamine) is repeated. The chain length of the glycosyl residue and, thus, also the molecular weight of the protein varies due to different numbers of repeats (Nimtz, M., et al., Eur. J. Biochem. 213 (1993) 39- 56) . In the case of EPO the triantennary glycosyl residues can contain a maximum of one repeat, whereas the tetraantennary glycosyl residues can have no more than two repeats. Biantennary glycosyl residues have no repeats.

Erythropoietin is a multiply glycosylated protein. Differently glycosylated forms and isoforms occur in nature as well as in biotechnological production due to variations in the glycosyl residues and the different degrees of sialidation. A high proportion of repeats also has a positive effect on the in vivo activity (see e.g. EP 0 409 113).

Erythropoietin consists of ten main isoforms. The term "isoform" refers to a group of EPO molecules which have an identical amino acid sequence and the same number of bound sialic acid residues. The isoforms have the same isoelectric point and differ with regard to the extent, complexity and antennarity of the glycosyl residues bound to the amino acid sequence. For example, the term "isoform 2 of EPO" encompasses a group of EPO molecules which have 14 sialic acid residues. Isoform 3 has 13 sialic acids and so on. In addition there are rare forms of EPO which have additional sialic acids which are not at the terminus. Thus, isoform 1 has 15 and isoform 1' has 16 sialic acids bound to the glycosyl residues.

One aspect of the invention is a method for preparing erythropoietin in high purity and with a distribution of erythropoietin isoforms, wherein the method comprises a combination of different chromatography steps in such a manner that the initial isoform distribution of erythropoietin is changed. The different chromatography steps of the method comprise a chromatography step with a stationary phase containing hydroxyapatite and at least one of the chromatography steps selected from i) dye affinity chromatography, ii) hydrophobic interaction chromatography, iii) anion exchange chromatography, iv) reversed phase chromatography, and/or v) gel permeation chromatography. In one embodiment of this aspect the erythropoietin isoforms with a reduced number of sialic acid residues are depleted or completely separated. Within this application the term "completely" means that the corresponding compound can no longer be detected by a given analytical method because the concentration/amount is below the detection limit of the method. In one embodiment the erythropoietin isoforms which have up to three, or up to four, or up to five (including five) sialic acid residues per erythropoietin molecule are depleted or completely separated. In one embodiment an erythropoietin is obtained in which the isoform mixture comprises isoforms with 6 to 15 sialic acid groups, or with 7 to 15 sialic acid groups.

In addition to the various glycosyl residues and isoforms further EPO forms exist. In case of de- N- EPO and de- O- EPO forms these are species that have not been completely posttranslationally glycosylated and are unglycosylated at serine residue 126 (de- O- EPO) or at asparagine residue 24 (de- N- EPO) .

Hydroxyapatite is an inorganic compound based on calcium phosphate having the empirical formula $Ca_5 (PO_4)_3 OH$. As a chromatographic stationary phase it is suitable for separating proteins, enzymes, immunoglobulins and nucleic acids. It occurs in nature primarily in bones and in dental enamel. The formation of hydroxyapatite is favored by a high pH value. Hydroxyapatite dissolves at an acidic pH value. The various binding mechanisms of hydroxyapatite are utilized in chromatographic separations where it primarily acts as an adsorbent but also has the properties of an ion exchange material. It is possible to form ionic bonds between the positively charged amino groups of a protein (e.g. lysine) and negatively charged phosphate groups of the stationary phase. In addition carboxyl groups of proteins (e.g. aspartate) can bind to the calcium ions of hydroxyapatite. A characteristic of the material is that complex binding can take place between the phosphate group of the stationary phase and calcium or magnesium ions in solution and the carboxyl groups of the protein (M. Kratzel, "Pharmazeutische Bioanalytik" Part 5, University of Vienna) .

Various hydroxyapatite materials are known. Erythropoietin binds to this matrix and is in one embodiment eluted at low phosphate concentrations.

A suitable column material for example is hydroxyapatite Ultrogel (BioSepra, Germany) or HA Biogel HT (Biorad, Germany). The chromatography is advantageously carried out at an approximately neutral pH. Hydroxyapatite embedded in an agarose matrix has a higher pressure resistance compared to pure hydroxyapatite (e.g. available as HA Ultrogel). This material has a heterogeneous size distribution of the particles between 50 $\mu$m and 160 $\mu$m with the main fraction between 70 $\mu$m and 90 $\mu$m. The surface is very rough and the particles are only spherical to a limited extent.

In a different embodiment the stationary phase containing hydroxyapatite for use in the method according to the invention consists of pure hydroxyapatite sintered under high temperature and high pressure (e.g. available as CHT - Ceramic Hydroxyapatite from BioRad). This is a pressure-resistant material and consists of particles which are relatively symmetrical and uniformly spherical and have a relatively smooth surface. The particles have a size of about 40 $\mu$m and a specific surface of about 40 m$^2$/g for CHT-type 1. The mean particle size is 20 $\mu$m - 30 $\mu$m. The CHT material type 2 was specially developed for larger molecules such as immunoglobulins. The specific surface of CHT-type 2 is 19 m$^2$/g and is, thus, about half that of CHT-type 1. The particles have a similar morphology to CHT-type 1. The particles also have a size of 40 $\mu$m. Thus, in one embodiment of the method according to the invention the stationary phase containing hydroxyapatite is a hydroxyapatite sintered under high temperatures and high pressures with a specific surface of about 40 m$^2$/g.

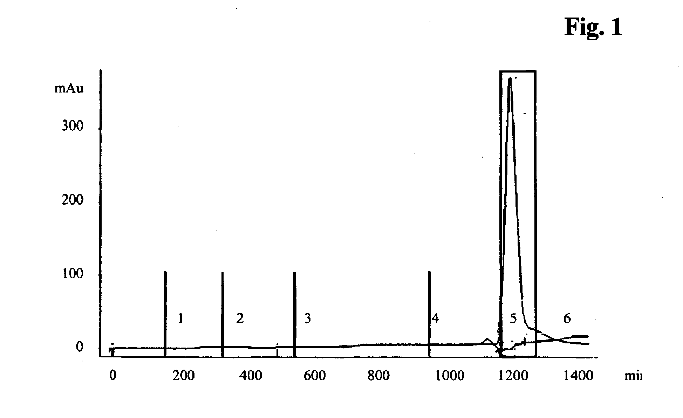

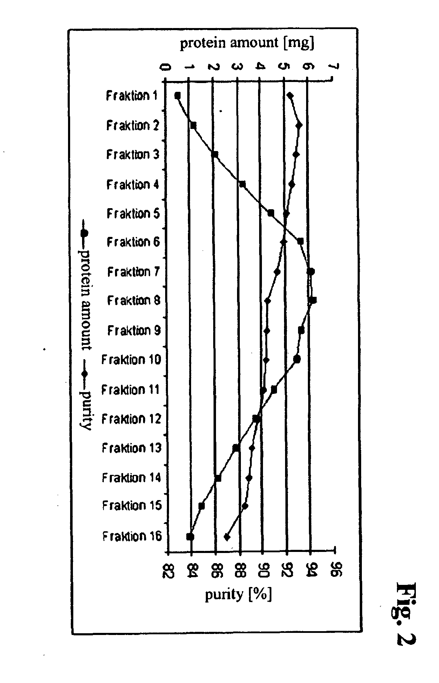

Figure 1 shows an example of a chromatogram of a chromatography of erythropoietin on a stationary phase containing hydroxyapatite (HA- Ultrogel) . The framed area is the fractionated product peak which was subdivided into fractions 1- 16. Fraction 17 is the peak shoulder which was collected as a separate fraction. The purity of the individual fractions decreases almost linearly over the peak (see Figure 2) .

The described different stationary phases containing hydroxyapatite were compared using the same chromatographic method for purifying erythropoietin which is described in Example 4.

**Table 1:** Summary of the data from the EPO chromatographies.

| Loading: 0.5 mg/ml column material | | | | |
|---|---|---|---|---|
| | **HA-Ultrogel** | **CHT-type** 1 | **CHT-type** 2 | **Fast Flow** |
| purity [%] | 99.5 - 99.8 | 99.3 - 99.8 | 99.8 - 99.9 | 99.8 - 99.9 |
| elution volume [ml] | 7 - 8.5 | 8 - 9 | 8 - 14 | 11.5 - 14 |
| product yield [%] | 49 - 59 | 47 - 54 | 44 - 54 | 53 |
| CHO protein content [ppm] | 24 - 1463 | 109 -1463 | 197 - complete depletion | 95 - 210 |
| Loading: 5 mg/ml column material | | | | |
| Loss of EPO [%] | 9.6 | 1.2 | 0.7 | 0.7 |
| purity of product peak [%] | 94.6 | 99.9 | 99.8 | 99.3 |
| elution volume [ml] | 23 | 32 | 23 | 60 |
| product yield [%] | 76.0 | 71.0 | 70.3 | 75.1 |
| CHO protein content [ppm] | 21484 | 8439 | 8339 | 11598 |

In one embodiment if the concentration of CHO protein was below 15 ng/ml, the sample was regarded as free of CHO protein. It has been found that the ceramic and the crystalline hydroxyapatite material are comparable when loaded with different amounts of erythropoietin, whereas the yield as well as the product quality were considerably reduced with a material based on agarose at a loading of 5 mg EPO/ml column material. Thus, in the separation with HA Ultrogel hydroxyapatite material almost 10% of the erythropoietin is lost during the separation.

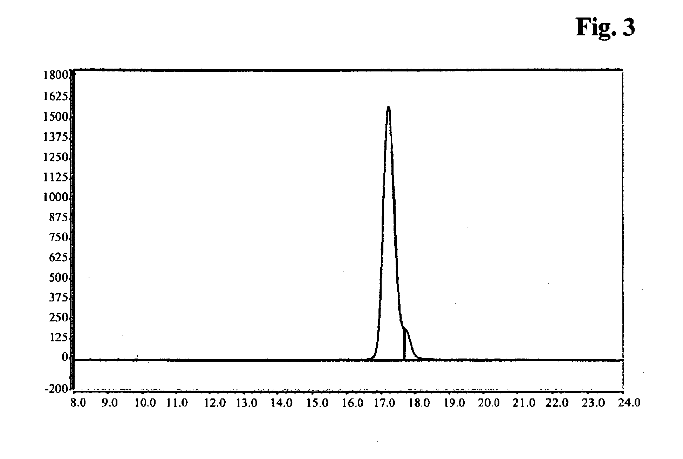

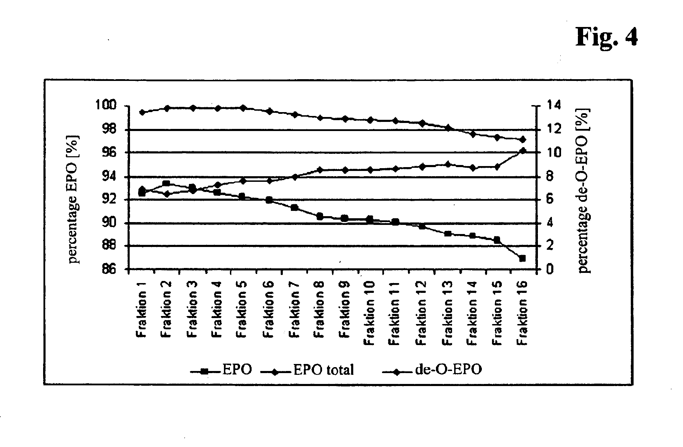

The analysis of the fractions containing erythropoietin by means of analytical reversed phase HPLC (RP-HPLC) shows that the product peak has a shoulder (Figure 3) in almost all fractions on the decreasing side of the erythropoietin peak which was identified as de-O-EPO. The proportion of de-O-EPO was determined semi quantitatively by drawing a perpendicular line in the RP-HPLC chromatograms and is shown together with the amount of EPO in Figure 4. The reason for the formation of this shoulder is that biantennary and triantennary glycosyl residue containing EPO forms are depleted among others in the hydroxyapatite chromatography. These forms in turn elute directly before de-O-EPO in the analytical RP-HPLC. As a result of this selective depletion the de-O-EPO shoulder stands out.

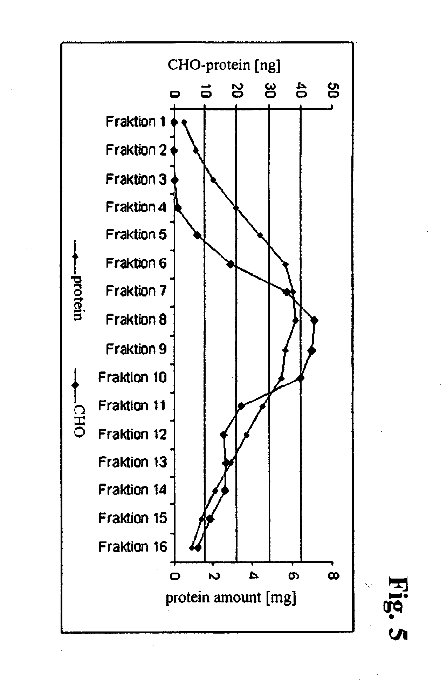

The protein-like impurities are distributed over the entire elution peak, but the front fractions contain a lower proportion of CHO host cell protein than the rear fractions (see Figure 5).

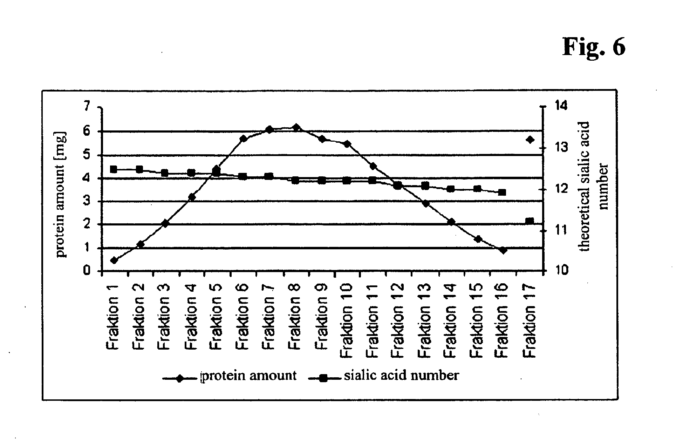

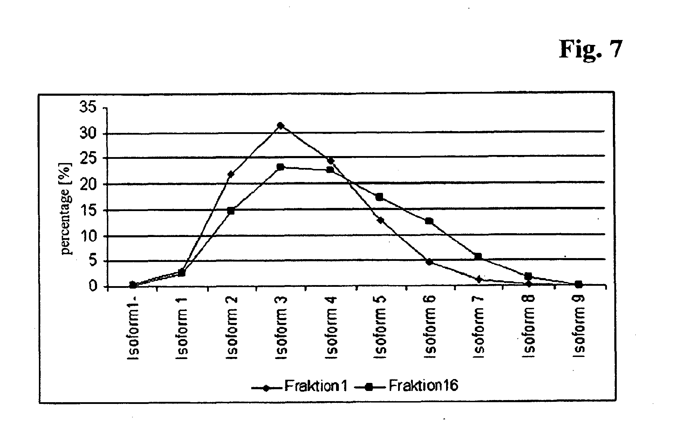

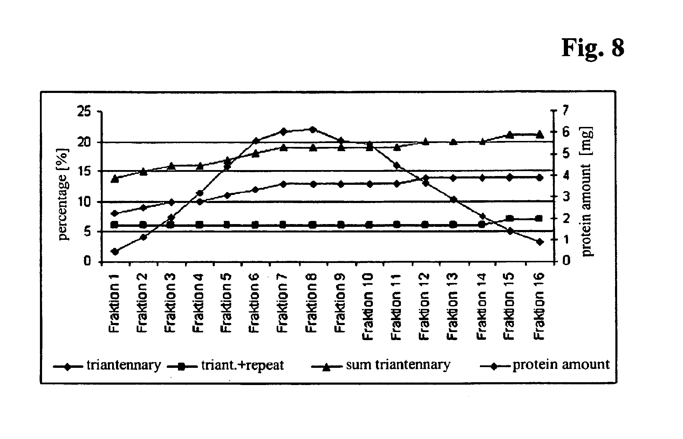

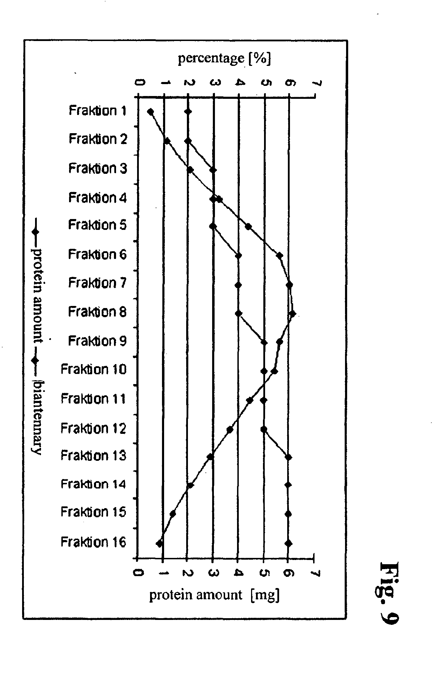

The hydrophobicity of erythropoietin increases due to the more hydrophobic EPO glycosyl forms in the later EPO-containing fractions. Chromatography on a stationary phase containing hydroxyapatite depletes especially basic isoforms i.e. isoforms containing less sialic acid. As shown in Figure 6, the number of sialic acid residues decreases uniformly over the erythropoietin-containing fractions. The basicity increases from isoform 1 to isoform 9. Whereas acidic isoforms are enriched in the first erythropoietin-containing fractions (fraction 1 to fraction 5), a considerable shift towards basic isoforms (Figure 7) is seen in the last erythropoietin-containing fractions (fraction 12 to fraction 16). Analysis of the glycosyl forms shows that hydrophilic EPO forms elute in the first fractions, which have a higher content of tetraantennary structures and more repeats. More hydrophobic EPO forms with fewer repeats or a higher proportion of triantennary structures elute later, the course of which is reciprocal to that of the tetraantennary glycosyl forms (Figure 8). The proportion of biantennary glycosyl forms increases steadily (Figure 9) whereas the increase is not uniform but rather in steps.

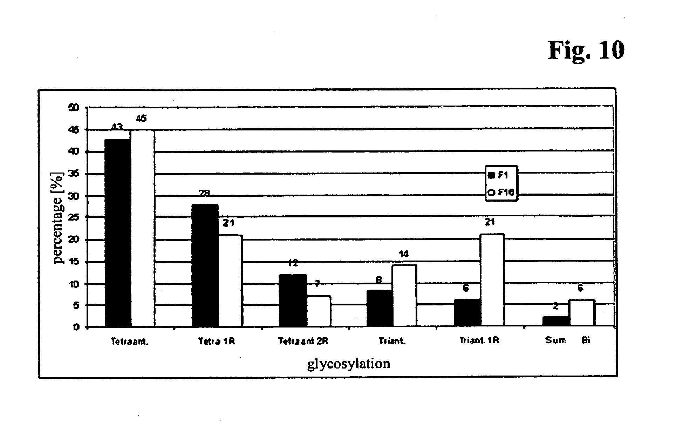

Figure 10 also illustrates the glycosyl form distribution over the product peak. Whereas tetraantennary glycosyl forms with a high repeat content are present in the first EPO-containing fractions (e.g. in F1, fraction 1), there is a high proportion of triantennary and biantennary glycosyl forms in the last EPO-containing fractions (e.g. in F16, fraction 16). Thus, it has been found that less glycosylated EPO forms are depleted and elute later in the chromatography on a stationary phase containing hydroxyapatite with a method according to the invention.

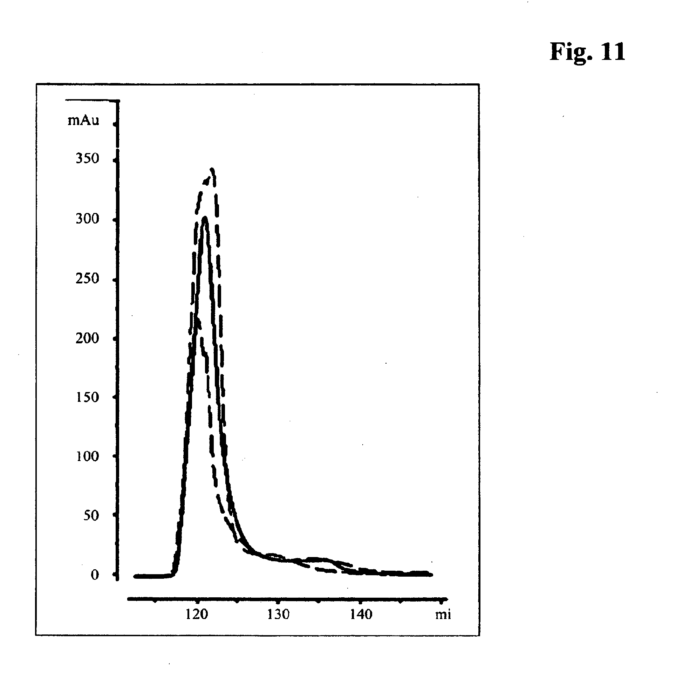

The content of CHO host cell proteins also increases in the terminal EPO-containing fractions. Consequently, a good separation and, thus, a depletion of CHO host cell proteins can be achieved with a chromatographic method having a better separation between EPO and CHO host cell proteins i.e. having a more pronounced shoulder containing the CHO host cell protein (see Figure 11).

This crystalline hydroxyapatite stationary phase is suited as the product peak is generally very flat and has a pronounced shoulder. In a further embodiment the solutions in the wash and elution step used in the chromatography contain a concentration of phosphate ions of 5 mM. It has been found that this phosphate ion concentration is advantageous for the purification of erythropoietin with a ceramic hydroxyapatite containing stationary phase as the amount of CHO host cell protein in EPO-containing fractions can be reduced/depleted to values below the quantification or detection limit of the corresponding analytical method by employing this phosphate concentration.

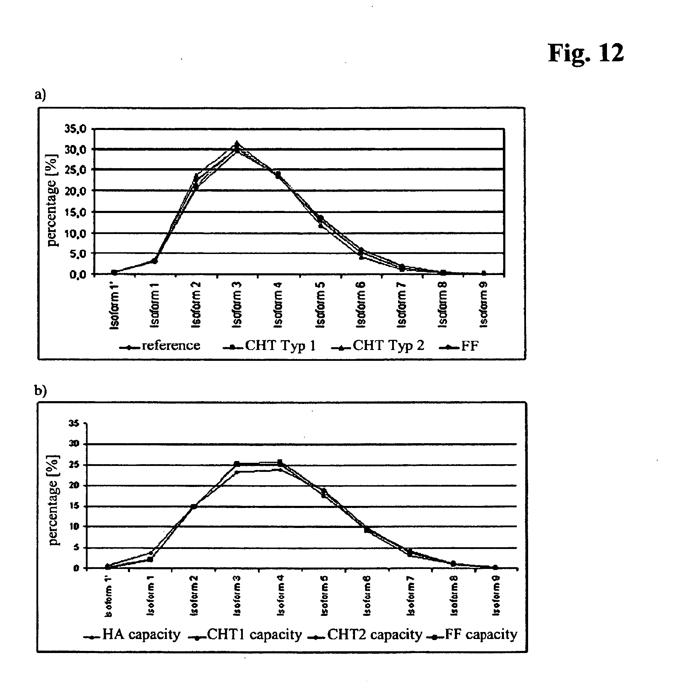

The isoform distribution of the erythropoietin obtained with the various stationary phases containing hydroxyapatite is shown in Figure 12. It has been found that neither the type of the hydroxyapatite containing stationary phase nor the loading has a major influence on the isoform distribution. A feature of ceramic and crystalline hydroxyapatite phases is that they deplete basic isoforms somewhat more than the agarose-based hydroxyapatite phases. In one embodiment the purity of the erythropoietin obtained with a method according to the invention is more than 99.3% determined by RP-HPLC. In one embodiment, optionally at a loading of 5 mg/ml, the purity of the EPO obtained is between 99.3% and 99.9% determined by RP-HPLC (% = area percent based on the RP-HPLC chromatogram). Differences between the stationary phases are also seen in the CHO host cell protein depletion/separation. The agarose-based hydroxyapatite phase as well as the crystalline hydroxyapatite phase are less effective than the ceramic hydroxyapatite phases. Hence in one embodiment of the methods according to the invention the stationary phase containing hydroxyapatite is a ceramic hydroxyapatite phase.

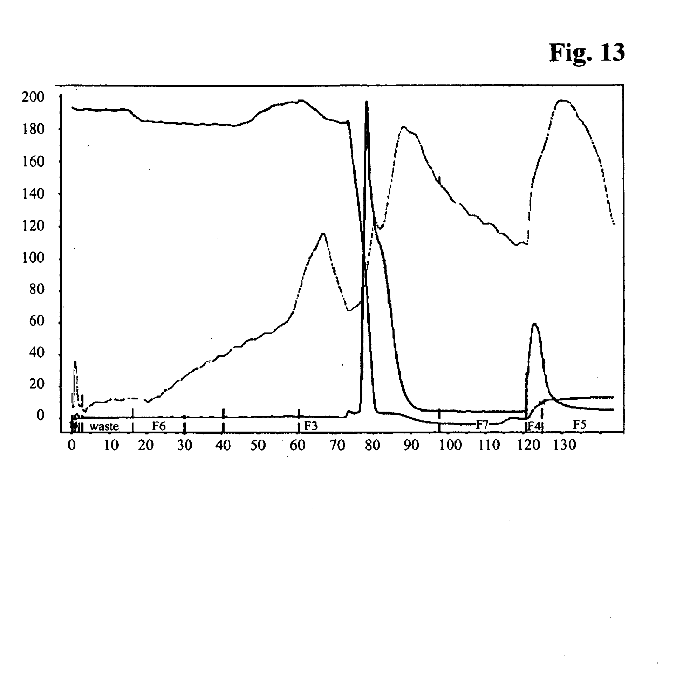

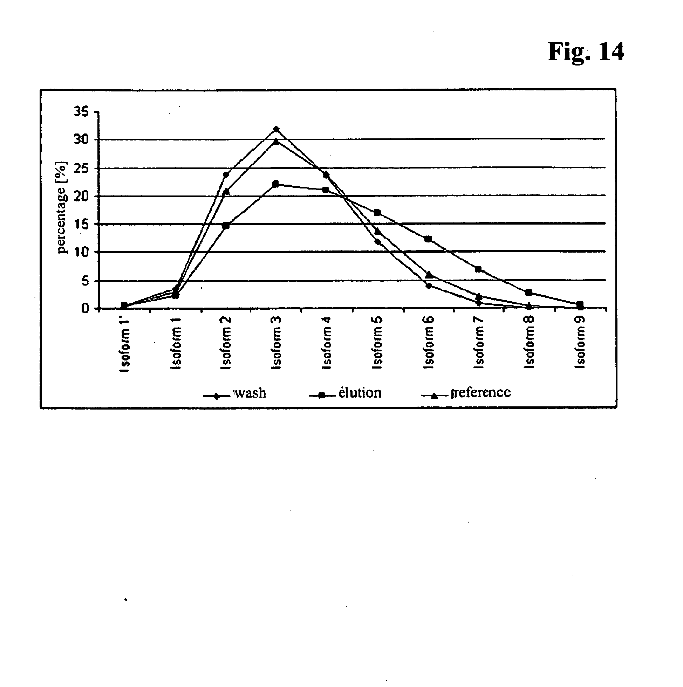

It has been found that the Calcium-ion concentration contained in the solutions used for the chromatography on a stationary phase containing hydroxyapatite has an pronounced influence on the purification and, therefore, also on the purity of the therewith obtained erythropoietin. When the Calcium-ion concentration is reduced in the solutions, it has turned out that most of the erythropoietin can be obtained from the stationary phase containing hydroxyapatite with the aid of a step-wise elution (i.e. by the step from solution three to solution four) (see Examples 4 and 8, and Figure 13). When the Calcium-ion concentration is increased after elution of the erythropoietin (with change to the fifth solution), CHO host cell protein and DNA elute from the stationary phase containing hydroxyapatite. The erythropoietin fractions obtained with the solution containing a reduced Calcium-ion concentration are far more acidic than the erythropoietin fractions eluted subsequently (see Figure 14). The decreasing side of the peak obtained with the increased Calcium-ion concentration is very flat i.e. it contains a lot of CHO host cell protein. In contrast the peak obtained with the reduced Calcium-ion concentration is very symmetrical.

The current invention comprises a method for purifying erythropoietin comprising at least one chromatography step using a stationary phase containing hydroxyapatite comprising

i)

> a) providing a first solution containing erythropoietin and Calcium-ions at a first concentration,
> b) providing a hydroxyapatite-containing stationary phase over which a volume of a second solution containing Calcium-ions at a second concentration has been passed (equilibration step),
> c) applying the solution of a) to the stationary phase obtained in b),

ii) passing a third solution containing a third concentration of Calcium-ions which is lower than that of the first and second solution over the stationary phase containing hydroxyapatite obtained in step i) (wash step), and

iii) passing a volume of a fourth solution which contains 0.5 mM Calcium-ions or less over the stationary phase containing hydroxyapatite from ii) and thereby detaching and recovering erythropoietin from the stationary phase containing hydroxyapatite and therewith obtaining a purified erythropoietin (elution step).

In one embodiment the first concentration of Calcium-ions is the same as the second concentration of Calcium-ions. In a further embodiment the second solution and the third solution contain 2-propanol. In a further embodiment the first and/or the second concentration of Calcium-ions is 5 mM. In a further embodiment the pH value of the solutions is pH 6.9 $\pm$ 0.2. In a further embodiment the 2-propanol content is 9% (v/v). In a further embodiment the third concentration of Calcium-ions is equal to or less than 0.1 mM, in one embodiment between 0.000001 mM and 0.1 mM. In another embodiment the fourth solution contains 0.1 mM Calcium-ions or less, in one embodiment between 0.000001 mM and 0.1 mM. In a further embodiment of from 4 to 7 column volumes of the second solution are applied to the column. In a further embodiment the volume applied to the column of the third solution is of from 0.5 to 2.5 column volumes. In a further embodiment only a small amount of erythropoietin is eluted/detached from the stationary phase in step ii). In a further embodiment this amount is less than 10%, in another embodiment less than 5%, and in a final embodiment less than 1% of the erythropoietin bound to the stationary phase. In a further embodiment the Calcium-ions are introduced into the solution as $CaCl_2$. In one embodiment of the method according to the invention the second solution contains 20 mM TRIS-HCl, 5 mM $CaCl_2$, 0.25 M NaCl, 9% (v/v) 2-propanol with a pH of 6.9 $\pm$ 0.2. In a further embodiment the third solution contains 20 mM TRIS-HCl, 0.25 M NaCl, 9% (v/v) 2-propanol with a pH of 6.9 $\pm$ 0.2. In a further embodiment the fourth solution contains 10 mM TRIS-HCl, pH 6.9 $\pm$ 0.2. The value of e.g. 9% (v/v) denotes that the solution is made by providing 9% 2-propanol and adding the prepared solution until the final volume is obtained.

The amount of tetraantennary glycosyl forms is increased in the fractions obtained with the fourth solution compared to the fractions obtained with the fifth solution. However, the fractions obtained with the fifth solution have a higher proportion of biantennary and triantennary glycosyl forms. It has also been found that with the fifth solution a higher proportion of de-O-EPO and de-N-EPO species are found in the fractions compared to the CHT-type 2 runs with $CaCl_2$ without modification.

A further aspect of the present invention is a method for producing erythropoietin comprising culturing a cell comprising an EPO-encoding nucleic acid in a suitable medium under suitable conditions and isolating erythropoietin from the culture medium or the cells. In one embodiment the cells are cultured in suspension. In a further embodiment the culture is carried out in a fermenter, in particular in a fermenter having a volume of 10 l - 50,000 l. The isolation of erythropoietin from the culture medium of the cell comprises the following steps:

> a) applying the cell culture supernatant or the supernatant of the disintegrated cell suspension to an affinity chromatography material and recovering/collecting the fractions containing erythropoietin,
> b) optionally applying the erythropoietin-containing fractions obtained in step a) to a hydrophobic interaction chromatography material and recovering/collecting the fractions containing EPO,
> c) applying the fractions containing EPO of a) or b) to a stationary phase containing hydroxyapatite and recovering/collecting the erythropoietin-containing fractions using a method according to the invention, and
> d) concentrating or/and applying the fractions containing EPO of c) to a reversed phase HPLC material.

Step a) of the purification method comprises applying the cell culture supernatant, which can optionally be pretreated, to an affinity chromatography material. In one embodiment the affinity chromatography material is one to which a blue dye has been covalently coupled. One example thereof is Blue Sepharose. In one embodiment, after eluting from the affinity chromatography material, the erythropoietin- containing eluate is applied to a hydrophobic interaction chromatography material if a culture medium with a serum content of equal to or more than 2% ($\geq$ 2%) (v/v) has been used. In one embodiment the interaction chromatography material is Butyl Sepharose. The eluate from step a) or, if used, step b) is applied to a stationary phase containing hydroxyapatite in step c) of the method and the eluate containing erythropoietin is recovered according to the method according to the present invention. In one embodiment the eluate is concentrated by means of exclusion chromatography, e.g. by membrane filtration, in which case the use of a medium, e.g. a membrane, with an exclusion size of 10 kDa has proven to be advantageous. The erythropoietin that can be obtained by the method according to the invention can contain α- 2, 3- linked or/and α- 2, 6- linked sialic acid residues.

In order to produce recombinant erythropoietin, the cell-free culture supernatant is isolated and subjected to the method according to the invention. If necessary a filtration to separate turbidities and/or a concentration can be additionally carried out before the purification process.

In the first step the dye chromatography removes contamination by proteases. In one embodiment a blue triazine dye such as Cibachron® Blue is used as the dye. Other triazine dyes are also suitable. The support material for the dye chromatography is uncritical but a polysaccharide-based support material is preferably used such as e.g. Sepharose, preferably Sepharose 6 Fast Flow. The column is equilibrated with buffer with a pH value of from 4.5 to 5.5, in one embodiment pH 4.8 - 5.2, in a further embodiment with acetate buffer or acetic acid. The chromatography is in one embodiment operated at temperatures of from 1°C to 10°C, in another embodiment at a temperature of about 5°C. The elution/recovering is in one embodiment by increasing the salt concentration at an acidic or neutral pH (in one embodiment at pH 5 to 7). At a basic pH, in one embodiment at pH 8.5 to 9.5, in another embodiment at pH 8.8 to 9.2, it is also possible to elute the erythropoietin without any major change in the salt concentration.

In the second step a chromatography on a hydrophobized support is carried out. Suitable adsorber materials for the hydrophobic chromatography are described for example in Protein Purification Methods, A practical Approach, Ed. Harris, E.L.V. and Angal, S., IRL Press, Oxford, England (1989), p. 224; and Protein Purification, Janson, J.C., Ryden, L., (ed.), VCH Publisher, Weinheim, Germany (1989), p. 207-226. The support material itself is uncritical and can be for example Sepharose, a copolymer of acrylic acid and methacrylic acid, or silica gel. It is important that hydrophobic groups, in one embodiment butyl groups, are covalently bound to the support. Suitable supports are commercially available (e.g. Butyl Toyopearl from Tosoh Haas, Germany, or Butyl Sepharose from Pharmacia, Germany). In one embodiment a butylated support is used. Other alkylated or arylated supports bind erythropoietin in some cases either irreversibly or result in a poorer separation. The elution in the hydrophobic chromatography takes place by lowering the salt concentration (e.g. with a gradient of 4 mol/l to 0 mol/l), by adding chaotropic agents such as iodide, perchlorate or thiocyanate, or by adding alcohols such as glycerol, ethylene glycol or 2-propanol. In one embodiment the hydrophobic chromatography is carried out at a neutral pH and in the presence of salt, in one embodiment of about 0.75 mol/l NaCl. It is also possible to carry out the hydrophobic chromatography in the presence of a low-molecular weight alcohol and in one embodiment in the presence of isopropanol (= 2-propanol). The concentration of the low molecular weight alcohol in the elution buffer is about twice to three times as high as in the equilibration buffer. In the wash buffer is the concentration of the low molecular weight alcohol about twice as high as in the equilibration buffer. About 10-15%, in one embodiment about 10% 2-propanol are added for the equilibration (loading of the chromatography material); about 25% to 35%, in one embodiment about 27% 2-propanol are added for the elution and 19% 2-propanol is added to the wash buffer (specified concentrations are also suitable for other alcohols, stated as volume %, v/v). The hydrophobic chromatography can be carried out in a wide temperature range of from 10°C to 40°C. However, it is advantageous to operate at a controlled temperature of 27 ± 2°C.

Next a separation on a stationary phase containing hydroxyapatite can be carried out according to method according to the invention.

The hydroxyapatite step can be followed by a reversed phase chromatography on a hydrophobized support as an optional step for purification. However, this is usually no longer necessary when using a hydroxyapatite chromatography according to the present invention and can therefore be omitted. In addition to the time saved this also reduces the costs and avoids the use of organic solvents which have to be subsequently separated. The following are for example suitable as chromatography materials for the reversed phase chromatography: Phenyl Sepharose and Octyl Sepharose (Pharmacia, Sweden), Butyl Toyopearl (Tosoh Haas, Germany) or Propyl-TSK (Merck, Germany). However, in this process step it is also advantageous to use supports which contain longer alkyl groups (e.g. $C_8$ or $C_{18}$). The column is in one embodiment equilibrated in a pH range between 2 and 7, in another embodiment at pH 2.5, and aqueous trifluoroacetic acid is in another embodiment used. A gradient from the equilibration buffer to an aqueous solution of a polar organic solvent such as for example acetonitrile is used for elution. The eluate is advantageously neutralized after the chromatography.

An anion exchange chromatography follows as the next step of the purification method according to the invention. In this step a DEAE Sepharose Fast Flow chromatography material is used as the column material in one embodiment. The equilibration takes place therewith at a pH value of from pH 6 to pH 9, in another embodiment at pH 7.5. Optionally after washing in one embodiment with an acidic solution (approximately of a pH value of 4.5), the elution is carried out in a neutral or slightly basic range (pH 6-9, in one embodiment pH 7.5 while increasing the ionic strength, in one embodiment with NaCl). Phosphate buffer is in one embodiment used as buffer.

In one embodiment the protein preparation is produced in batches of from 0.1 g to 10 g. It has turned out that EPO can be sufficiently purified for therapeutic use after culture in a medium which in one embodiment is free of natural mammalian proteins if a hydrophobic chromatography is carried out as a second step after the affinity chromatography on a dye (EP 1 394 179). It is not necessary to add protease inhibitors (e.g. $CuSO_4$) before the chromatographic

purification as described by Nobuo, I., et al., J. Biochem. 107 (1990) 352-359 or in WO 86/07494. The hydrophobic chromatography is in one embodiment carried out on an alkylated ($C_4$-$C_{18}$) or arylated (in one embodiment phenylated or benzylated) support. A butylated support is particularly used in another embodiment.

"Completely free of natural mammalian proteins" denotes herein that the preparation contains no such protein in detectable amounts. The preparation is completely free from mammalian proteins added intentionally which are not derived from the host cell and are otherwise usually added to the culture medium to maintain and improve cell growth as well as to optimize the yield. Natural mammalian proteins are understood as mammalian proteins from natural sources such as from human or from animal, but not recombinant mammalian proteins which have for example been produced in prokaryotes such as E. coli.

A further aspect of the present invention is a method for producing erythropoietin covalently linked to one poly (ethyleneglycol) polymer (mono-PEGylated erythropoietin) comprising the following steps:

a) conjugating erythropoietin to poly(ethyleneglycol) (PEGylation of erythropoietin) by using an activated PEG reagent having a molecular weight between 20 kDa and 40 kDa,

b) purifying the PEGylated erythropoietin obtained in step a) with two successive cation exchange chromatography steps each of them employing the same stationary phase, but different elution methods,

c) isolating the erythropoietin conjugated to PEG (poly (ethyleneglycol) ) from the second stationary phase.

This method is particularly suitable for purifying PEGylated EPO which has been produced recombinantly and which has been subsequently chemically PEGylated. In the first step of the method is the erythropoietin PEGylated. The poly(ethyleneglycol) polymer (PEG) which is used in this reaction has a molecular weight of approximately 20 kDa to 40 kDa (the term "molecular weight" refers in this connection to the average molecular weight of poly(ethyleneglycol) because PEG is a polymeric compound which is not obtained with a defined molecular weight but rather has a molecular weight distribution). The term "approximately" means that some PEG molecules will have a higher molecular weight and some will have a lower molecular weight i.e. the term "approximately" means that it is a molecular weight distribution in which 95% of the PEG molecules have a molecular weight of +/- 10% of the stated molecular weight.

The term "PEGylation" refers to a covalent linkage between a poly (ethyleneglycol) molecule and the N-terminus or a lysine side chain of a polypeptide. The PEGylation of proteins is described inter alia in Veronese, F.M., Biomaterials 22 (2001) 405- 417. Poly (ethyleneglycol) can be linked with proteins by means of various functional groups and with various molecular weights (Francis, G.E., et al., Int. J. Hematol. 68 (1998) 1- 18; Delgado, C., et al., Crit. Rev. Ther. Drug Carrier Systems 9 (1992) 249- 304) . The covalent linkage of poly (ethyleneglycol) and erythropoietin can be carried out in aqueous solution e.g. as reported in WO 00/44785, in one embodiment using NHS- activated linear or branched poly (ethyleneglycol) (PEG) molecules having a molecular weight between 5 kDa and 40 kDa. The PEGylation reaction can also be carried out as a solid phase reaction (Lu, Y., et al., Reactive Polymers 22 (1994) 221- 229) . Selective N- terminal PEGylated proteins can be obtained according to WO 94/01451 or Felix, A.M., et al., ACS Symp. Ser. 680 (poly (ethyleneglycol) ) (1997) 218- 238.

The following examples, sequence listing and figures are provided to aid the understanding of the present invention, the true scope of which is set forth in the appended claims. It is understood that modifications can be made in the procedures set forth without departing from the spirit of the invention.

## Description of the Sequence Listing

**SEQ ID NO: 1** Human erythropoietin with an amino acid sequence of 165 residues.

**SEQ ID NO: 2** Human erythropoietin with an amino acid sequence of 166 residues.

## Description of the Figures

| | |
|---|---|
| **Figure 1** | Exemplary chromatogram for a chromatography of erythropoietin with a stationary phase containing hydroxyapatite (HA- Ultrogel) . |
| **Figure 2** | Plot of the purity of individual erythropoietin- containing fractions. |
| **Figure 3** | Analytical chromatography of a fraction containing erythropoietin by means of analytical reversed phase HPLC. |
| **Figure 4** | Diagram of the percentage of de- O- EPO and EPO in the erythropoietin- containing fractions. |
| **Figure 5** | Distribution of protein- like impurities in the erythropoietin- containing fractions. |

**Figure 6**     Plot of the sialic acid count of the erythropoietin- containing fractions.

**Figure 7**     Distribution of the acidic and basic isoforms in the erythropoietin- containing fractions.

**Figure 8**     Distribution of the triantennary glycosyl forms in the erythropoietin- containing fractions.

**Figure 9**     Distribution of the biantennary glycosyl forms in the erythropoietin- containing fractions.

**Figure 10**    Overview of the distribution of glycosyl forms in the erythropoietin- containing fractions.

**Figure 11**    Comparison example of the shoulder in an erythropoietin- containing peak.

**Figure 12**    Isoform distribution of erythropoietin obtained with different stationary phases containing hydroxyapatite.

**Figure 13**    Chromatogram obtained using the method according to the invention.

**Figure 14**    Comparison of the isoform distribution in the different chromatographic methods.

## Materials and Methods

### Buffer for the chromatography:

All solutions were prepared from stock solutions. If not stated otherwise the pH of all buffers was adjusted with 1 M NaOH or 1 M HCl. Type II superpure water (MilliQ- water) was always used.

### Stock solution (in water):

1 mol/l potassium phosphate buffer, pH 6.9 $\pm$ 0.2,

1 mol/l tris (hydroxyaminomethane) buffer (TRIS- HCl) , pH 6.9 $\pm$ 0.2,

5 mol/l sodium chloride,

1 mol/l calcium chloride,

10 mol/l sodium hydroxide.

### Standard chromatography buffer:

| | |
|---|---|
| standard wash buffer: | 20 mM TRIS-HCl, 5 mM CaCl$_2$, pH 6.9 $\pm$ 0.2 |
| packing buffer HA Ultrogel/Fast Flow: | 20 mM TRIS-HCl, 5 mM CaCl$_2$, pH 6.9 $\pm$ 0.2 |
| packing buffer CHT: | 200 mM potassium phosphate, pH 9-10 |
| regeneration buffer 1 HA Ultrogel: | 200 mM potassium phosphate, 0.1 mM CaCl$_2$, pH 6.9 $\pm$ 0.2 |
| regeneration buffer 1 CHT, Fast Flow: | 200 mM potassium phosphate, pH 6.9 $\pm$ 0.2 |
| alkaline regeneration: | 0.5 M NaOH |
| equilibration buffer: | 20 mM TRIS-HCl, 5 mM CaCl$_2$, 0.25 M NaCl, 9 % (v/v) 2-propanol, pH 6.9 $\pm$ 0.2 |
| wash buffer: | 10 mM TRIS-HCl, 5 mM CaCl$_2$, pH 6.9 $\pm$ 0.2 |
| elution buffer: | 10 mM TRIS-HCl, 0.5 mM CaCl$_2$, 10 mM potassium phosphate, pH 6.9 $\pm$ 0.2 |

### Buffers for analytical determinations

analytical RP-HPLC:

eluent A: 0.1% TFA (trifluoroacetic acid), water eluent B: 0.1% TFA, 70% acetonitrile, water
protein determination A$_{280}$:

10 mM Na/K phosphate, 0.1 mol/l NaCl, pH 7.5 $\pm$ 0.2

### Protein determination

A quartz cuvette with a path length of 1 cm was used. The samples were diluted such that the determined absorbance was between 0.2 and 1.0 AU. If a dilution was required the samples were diluted independently of one another and measured in a triplicate determination. Undiluted samples were determined with a single measurement. The erythropoietin final product buffer was used for sample dilution and for the blank determination.

The measurement was carried out at 280 nm. An erythropoietin reference standard sample was measured at the beginning as a system control. The actual samples were adjusted prior to the analysis to an average protein concentration of 1.86 mg/ml to 2.05 mg/ml. The protein concentration was calculated on the basis of the following formula:

$$c = \frac{A_{280}}{1.25^{1)}} \cdot F^{2)} [mg/ml]$$

1) extinction coefficient of a 0.1% solution: $\varepsilon = 1.25 ml \cdot (mg \cdot cm)^{-1}$

2) dilution factor of the sample

The amount of protein is given by the following formula:

$$m[g] = c[mg/ml] \cdot V[L]$$

Analytical RP-HPLC

In the RP-HPLC (reversed phase-high performance liquid chromatography) all samples were diluted with eluent A to a maximum concentration of 0.12 mg/ml. The protein concentration was determined by photometric measurements at 280 nm. Four samples of 100% eluent A were injected prior to the sample analysis. This ensured that the column was completely equilibrated. The erythropoietin reference standard was analyzed at the start and at the end of the sample sequence. In order to avoid deviations, the chromatograms were analyzed by a standardized integration method. Each of the peaks was separated from one another by drawing a line perpendicular to the base.

Determination of the CHO protein content/host cell protein content

The CHO cell proteins were determined by means of an ELISA test (enzyme-linked immunosorbent assay). Herein a sandwich assay based on the streptavidin/biotin technology was used. A streptavidin-coated microtiter plate was used for the assay. An antibody which was directed against CHO cell protein was bound to the plate by means of biotin. The sample solution to be analyzed was added by pipette at a CHO protein concentration of 15-150 ng/ml. If the concentration was below 15 ng/ml, the sample was regarded as free of CHO protein. If the concentration was over 150 ng/ml, the test was repeated with a higher dilution.

The results were stated as CHO protein [ppm].

$$\frac{\frac{ng}{ml} CHO}{\frac{mg}{ml} EPO} = ppm$$

Determination of the isoform distribution

The isoform distribution was determined by means of capillary electrophoresis. For the analysis the sample was diafiltered in water. Subsequently the capillary was rinsed with an electrolyte solution followed by sample application of the diluted samples into the capillary. The separation took place by applying a high voltage of 25,000 V. The mobile buffer had an excess of positive ions which resulted in an electroosmotic flow. Use of a quartz capillary enabled a photometric detection of the proteins in the capillary and a quantitative determination by means of the peak area.

Sugar analysis

For sugar analysis an enzymatic test procedure was used. In this procedure the N-glycosidically-linked oligosaccharides of erythropoietin were cleaved by the enzyme N-glycosidase. In addition the sialic acids were removed from the oligosaccharides with the aid of the enzyme neuramidase. The N-oligosaccharides that were obtained were separated and analyzed using an anion exchanger. An erythropoietin reference standard which was treated in exactly the same manner as the samples was measured during the series of tests. The following sugar structures were detected: biantennary, triantennary, triantennary 1 repeat, tetraantennary, tetraantennary 1 repeat, tetraantennary 2 repeats.

Exclusion chromatography (SE-HPLC)

For SE-HPLC the chromatography column was equilibrated with three to five column volumes (CV) buffer to obtain a uniform baseline without additional peaks before the separation was started. The samples were diluted to a concentration of 0.2 mg/ml and injected into the SE-HPLC. The peaks were integrated according to standard methods. The peaks were separated from one another by dropping perpendiculars.

DNA determination

Biotin was bound to a microtiter plate. A reaction mixture of streptavidin, single-stranded DNA and biotinylated single-stranded DNA binding protein was added. The binding protein was able to bind DNA and was biotinylated. In this manner it was possible to specifically remove the DNA from the sample mixture. The streptavidin bound the biotin on the microtiter plate as well as the biotin which was coupled to the single-stranded DNA binding protein. A DNA-specific antibody which was coupled to urease was added to this total complex. Addition of urea resulted in a hydrolysis of the urea which caused a local change in the pH. This change can be detected as an altered surface potential. The change in the surface potential was proportional to the amount of bound DNA. Single stranded DNA was obtained by proteinase K digestion and denaturation with SDS.

Peptide Map

Incompletely glycosylated erythropoietin species such as de-O-EPO and de-N-EPO can be quantitatively determined by peptide mapping. For this purpose the erythropoietin molecule was cleaved into peptides by the endoproteinase Lys-C and these peptides were separated by HPLC. The peptide pattern obtained was compared with a reference standard. The results obtained were compared with the standard with regard to peak size, peak appearance and retention time.

## Example 1

### Recombinant production of erythropoietin in CHO cells

EPO is produced in CHO cells by a batch method. The fermenter is inoculated with a pre- culture and after about five days the fermenter content is harvested. Intact CHO cells and cell fragments were removed from the fermentation supernatant by centrifugation. The pH of the cell- free culture supernatant was adjusted with acetic acid (1 mol/l) to pH 5.0- 5.2 and the pH- adjusted solution was subsequently filtered at 1- 9°C. A serum- free medium was used as the culture medium which consisted of the base medium DME (HG) HAM's F- 12 modified (R5) (GRH Biosciences/ Hazleton Biologics, Denver, USA, Order No. 57- 736) , sodium hydrogen carbonate, L- (+) glutamine, D- (+) glucose, recombinant insulin, sodium selenite, diaminobutane, hydrocortisone, iron (II) sulfate, asparagine, aspartic acid, serine and polyvinyl alcohol.

## Example 2

### Blue Sepharose chromatography

Blue Sepharose (Pharmacia) consists of Sepharose beads to which the dye Cibachron® blue is covalently bound. Erythropoietin binds to this support at low ionic strength and neutral to acidic pH values. The erythropoietin is eluted by increasing the ionic strength and the pH value.

The chromatography column (Amicon P440 x 500, Amicon, GB) is filled with 60-80 l Blue Sepharose and regenerated with 0.5 N NaOH. Subsequently the column is equilibrated with about three column volumes (CV) acetate buffer. The cell-free culture supernatant adjusted to pH 5 is absorbed to the column at a temperature of 10 +/- 5°C and a flow rate of 800-1400 ml/min. The column is rewashed at the same flow rate and 5 +/- 4°C with about 1 CV wash buffer 1 followed by about 2 CV wash buffer 2. Subsequently the column is eluted with about 3 CV elution buffer. The entire protein peak is collected (about 30-60 l), adjusted to pH 6.9 with HCl and stored at 5 +/- 4°C until further processing.

The product solution is concentrated in this chromatography step and a purity of about 40-50% is achieved.

| | |
|---|---|
| equilibration buffer: | 20 mM Na acetate, 5 mM $CaCl_2$, 0.1 M NaCl, pH 5.0 $\pm$ 0.2 |
| wash buffer 1: | 20 mM Na acetate, 5 mM $CaCl_2$, 0.25 M NaCl, pH 5.0 $\pm$ 0.2 |
| wash buffer 2: elution buffer: | 20 mM TRIS-HCl, 5 mM $CaCl_2$, pH 6.5 $\pm$ 0.3 100 mM TRIS-HCl, 5 mM $CaCl_2$, 1 M NaCl, pH 9.0 $\pm$ 0.2 |

## Example 3

### Butyl Toyopearl chromatography (hydrophobic chromatography)

Butyl Toyopearl (Tosoh Haas) is a support on which butyl residues are covalently bound to the surface. The erythropoietin binds to this matrix and is eluted with a buffer containing 2-propanol.

After the protein had bound to the butyl matrix in an equilibration buffer which contains 10% 2-propanol, the erythropoietin was eluted by a gradient consisting of aqueous buffer solution and 50% 2-propanol. The elution begins at about 20% 2-propanol.

The chromatography column (Pharmacia BPG 300/500) is filled with 30-40 l Butyl Toyopearl and regenerated with 4 M guanidine-HCl and 0.5 N NaOH. Subsequently the column is equilibrated with at least three CV equilibration buffer. The eluate of the Blue Sepharose column is adjusted to 10% 2-propanol and absorbed to the column at a temperature of 27 $\pm$ 2°C and a flow rate of 800-1200 ml/min. The column is rewashed with about one CV equilibration buffer at the same temperature and flow rate and then with about two CV wash buffer. Subsequently the erythropoietin is eluted with about three CV elution buffer. The entire protein peak is collected (about 10-18 l), immediately diluted three-fold with dilution buffer and stored at 15°C. A purity of about 90% is achieved in this chromatography.

| | |
|---|---|
| equilibration buffer: | 20 mM TRIS-HCl, 5 mM $CaCl_2$, 0.75 M NaCl, 10% 2-propanol, pH 6.9 $\pm$ 0.2 |
| wash buffer: | 20 mM TRIS-HCl, 5 mM $CaCl_2$, 0.75 M NaCl, 19% 2-propanol, pH 6.9 $\pm$ 0.2 |
| elution buffer: | 20 mM TRIS-HCl, 5 mM $CaCl_2$, 0.75 M NaCl, 27% 2-propanol, pH 6.9 $\pm$ 0.2 |
| dilution buffer: | 20 mM TRIS-HCl, 5 mM CaCl2, pH 6.9 $\pm$ 0.2 |
| first solution: | 20 mM TRIS-HCl, 5 mM CaCl2, 0.25 M NaCl, 9% (v/v) 2-propanol, pH 6.9 $\pm$ 0.2 |

## Example 4

### Chromatography on stationary phases containing hydroxyapatite

The same basic method steps have been used in the separation on each of the stationary phases containing hydroxyapatite in order to achieve a good comparability based on the current process conditions. The sequence was:

regeneration - equilibration - separation - regeneration.

The buffers were adapted to the manufacturer's instructions for the regeneration.

**Table 2:** Regeneration - equilibration.

| Regeneration | | | |
|---|---|---|---|
| | HA Ultrogel | Fast Flow | CHT type 1 and type 2 |
| step 1 | regeneration buffer 1 HA Ultrogel | regeneration buffer 1 CHT, Fast Flow | regeneration buffer 1 CHT type 1, 2 |
| step 2 | alkaline regeneration | alkaline regeneration | alkaline regeneration |
| step 3 | -- | -- | regeneration buffer 1 CHT type 1, 2 |
| step 4 | equilibration | equilibration | equilibration |

**Table 3:** Separation.

| Separation | |
|---|---|
| step 1 | absorption |
| step 2 | wash 1 (equilibration buffer) |
| step 3 | wash 2 (wash buffer) |
| step 4 | elution |

The chromatography parameters for the separations are shown in the following Table 4. The same were used for all materials.

**Table 4:** Chromatography parameters.

| process step | Buffer | volume |
|---|---|---|
| reg. buffer 1 | regeneration buffer 1 | 2.3 CV (1.79 - 2.69 CV) |
| regeneration | regeneration buffer basic (NaOH) | 2.15 CV (1.79 - 2.69 CV) |
| washing | standard wash buffer | 6.5 CV (5.97 - 8.95 CV) |
| equilibration | equilibration buffer | 5.75 CV |
| absorption | solution to be separated | varies depending on the protein concentration loading: 0.5 - 5 mg/ml |
| wash 1 | equilibration buffer | 1.3 CV (0.77 - 1.15 CV) |
| wash 2 | wash buffer | 2.3 CV (1.54 - 2.31 CV) |
| elution | elution buffer | about 2.6 CV (as required) |

The peaks were collected on the basis of the UV absorption signal. The collection was started at 15 mAU and stopped after the respective peak maximum has been passed at 40 mAU. The regeneration was carried out after the separation. The peaks of the elution and regeneration steps were collected and stored at -20°C. The cut limits for the regeneration were at 15 mAU in the increasing flank to 15 mAU in the decreasing flank. The elution flow rate was varied between 0.35 cm/min and 1.7 cm/min in the individual steps.

Standard solutions:

standard wash buffer:  20 mM TRIS-HCl, 5 mM $CaCl_2$, pH 6.9 $\pm$ 0.2
packing buffer HA Ultrogel/Fast Flow:  20 mM TRIS-HCl, 5 mM $CaCl_2$, pH 6.9 $\pm$ 0.2
packing buffer CHT:  200 mM potassium phosphate, pH 9-10
regeneration buffer 1 HA Ultrogel:  200 mM potassium phosphate, 0.1 mM $CaCl_2$, pH 6.9 $\pm$ 0.2
regeneration buffer 1 CHT, Fast Flow:  200 mM potassium phosphate, pH 6.9 $\pm$ 0.2
alkaline regeneration:  0.5 M NaOH
equilibration buffer:  20 mM TRIS-HCl, 5 mM $CaCl_2$, 0.25 M NaCl, 9 % (v/v) 2-propanol, pH 6.9 $\pm$ 0.2
wash buffer:  10 mM TRIS-HCl, 5 mM $CaCl_2$, pH 6.9 $\pm$ 0.2
elution buffer:  10 mM TRIS-HCl, 0.5 mM $CaCl_2$, 10 mM potassium phosphate, pH 6.9 $\pm$ 0.2

## Example 5

### Hydroxyapatite Ultrogel chromatography

Hydroxyapatite Ultrogel (BioSepra) consists of hydroxyapatite which is incorporated in an agarose backbone in order to improve its mechanical and hydrodynamic properties. The erythropoietin binds to this matrix and is eluted at a lower phosphate concentration than most of the proteinaceous impurities.

The chromatography column (Amicon P440 x 500 or equivalent) is packed with 30-40 1 hydroxyapatite Ultrogel and regenerated with 0.5 N NaOH. Subsequently the column is equilibrated with at least four CV equilibration buffer. The erythropoietin solution to be purified is adsorbed to the column at a temperature of about 15°C and a flow rate of 500-1200 ml/min. The column is rewashed with about one CV equilibration buffer and then with about two CV wash buffer at the same temperature and flow rate. It is subsequently eluted with about 3 CV elution buffer. The entire protein peak is collected (about 10 - 18 1) and stored at 15°C until further processing. A purity of more than 95% is achieved in this chromatography.

| | |
|---|---|
| equilibration buffer: | 20 mM Na acetate, 5 mM $CaCl_2$, 0.1 M NaCl, pH 5.0 ± 0.2 |
| wash buffer 1: | 20 mM Na acetate, 5 mM $CaCl_2$, 0.25 M NaCl, pH 5.0 ± 0.2 |
| wash buffer 2: | 20 mM TRIS-HCl, 5 mM $CaCl_2$, pH 6.5 ± 0.3 |
| elution buffer | 100 mM TRIS-HCl, 5 mM $CaCl_2$, 1 M NaCl, pH 9.0 ± 0.2 |

### Example 6

**Hydroxyapatite Fast Flow chromatography**

This material was supplied by the manufacturer as a solid. The appropriate amount of material was weighed and suspended in standard wash buffer. The powder was incubated in the buffer for a few hours in order to wet the entire surface of the particles. A thin gel slurry (about 20% (v/v)) was used for packing. The column was packed without pressure by allowing the gel material to sediment for two days. After the gel had completely sedimented, the column was rinsed with five CV at a flow rate of 0.5 ml/min (0.64 cm/min). Subsequently the column was regenerated.

The chromatography was carried out as described in example 4.

### Example 7

**Hydroxyapatite CHT chromatography**

The ceramic materials (CHT type 1 and 2) were supplied by the manufacturer as a solid. The amount to be filled was weighed and suspended in packing buffer CHT type 1, 2. The CHT was incubated for a few hours in packing buffer in order to wet all areas of the material. A thin gel slurry (about 20% (v/v)) was used for packing and the entire slurry was filled into the column and packed at a flow rate of 1 ml/min (1.3 cm/h). After the gel had completely sedimented, the column was regenerated.

The chromatography was carried out as described in example 4.

### Example 8

**Hydroxyapatite CHT chromatography according to the invention without $CaCl_2$**

The column was packed as described in example 7 and the chromatography was carried out as described in example 4.

In contrast to example 4 the following solutions were used:

| | |
|---|---|
| second solution: | 20 mM TRIS-HCl, 5 mM $CaCl_2$, 0.25 M NaCl, 9% (v/v) 2-propanol, pH 6.9 ± 0.2 |
| third solution: | 20 mM TRIS-HCl, 0.25 M NaCl, 9% (v/v) 2-propanol, pH 6.9 ± 0.2 |
| fourth solution: | 10 mM TRIS-HCl, pH 6.9 ± 0.2 |
| fifth solution: | 10 mM TRIS-HCl, 5 mM $CaCl_2$, 10 mM potassium phosphate, pH 6.9 ± 0.2 |

### Example 9

**Reversed phase HPLC (RP-HPLC)**

The RP-HPLC material e.g. Vydac C4 (Vydac, USA) consists of silica gel particles whose surface carries C4 alkyl chains. The erythropoietin binds to this matrix as a result of hydrophobic interactions and is selectively eluted with an acetonitrile gradient in dilute trifluoroacetic acid.

The preparative HPLC was carried out with a Merck Prepbar 100 separation system (or equivalent) at a temperature of 22 ± 4°C. The separation column (100 mm x 400 mm, 3.2 1) was packed with Vydac C4 material. Before use the column was regenerated by applying several times a gradient of buffer A to 100% solvent and is subsequently equilibrated with buffer A. The eluate of the hydroxyapatite column was acidified to about pH 2.5 with trifluoroacetic acid and sterilized by filtration. Subsequently the erythropoietin is absorbed to the column at a temperature of 22 ± 4°C and a flow rate of 250 - 310 ml/min. The column was eluted with a linear gradient of buffer A to buffer B at the same temperature and flow rate. The elution peak is collected in fractions. The eluate is immediately neutralized by adding four volumes HPLC dilution buffer.

Fractions which have a purity of at least 99% in the analytical HPLC are pooled (pool volume about 4-6 1). Trace impurities are separated in this chromatography and a purity of more than 99% is achieved.

| | |
|---|---|
| buffer A: | 0.1 % trifluoroacetic acid in water |
| buffer B: | 80% acetonitrile, 0.1 % trifluoroacetic acid in water |
| HPLC dilution buffer: | 10 mM Na/K phosphate, pH 7.5 ± 0.2 |

## Example 10

### DEAE Sepharose FF chromatography

DEAE Sepharose Fast Flow (Pharmacia) consists of DEAE groups which are covalently bound to the surface of Sepharose beads. The erythropoietin binds to this matrix as a result of ionic interactions and is eluted by increasing the ionic strength.

The chromatography column (Amicon P90 x 250 or equivalent) was filled with 100-200 ml gel per g of erythropoietin in the applied sample and regenerated with 0.5 M NaOH. Subsequently the column is equilibrated with 100 mM Na/K buffer, pH 7.5 and afterwards with at least 12 CV equilibration buffer. The eluate of the HPLC column is absorbed to the column at a temperature of 5 ± 4°C and a flow rate of about 150 ml/min. The column was washed with at least five CV equilibration buffer and then with about ten CV wash buffer at the same temperature and flow rate. Subsequently the column was again washed with about ten CV equilibration buffer and the erythropoietin was eluted with about seven CV elution buffer. The entire protein peak is collected (about 2-5 1), sterilized by filtration and dispensed.

In this chromatography the solvent from the HPLC step is separated and trace impurities are removed. The purity is more than 99%.

| | |
|---|---|
| equilibration buffer: | 10 mM Na/K phosphate, pH 7.5 ± 0.2 |
| wash buffer: | 30 mM Na acetate, pH 4.5 ± 0.1 |
| elution buffer: | 10 mM Na/K phosphate, 80 mM NaCl, pH 7.5 ± 0.2 |

## Claims

1. An in vitro method for purifying erythropoietin comprising

   a) providing a solution containing i) erythropoietin and ii) Calcium-ions at a first concentration comprising 20 mM TRIS-HCl, 5 mM CaCl$_2$, pH 6.9 ± 0.2,
   b) providing a chromatography column containing a ceramic hydroxyapatite-containing stationary phase,
   c) applying a solution containing Calcium-ions at a second concentration to the column of b) comprising 20 mM TRIS-HCl, 5 mM CaCl$_2$, 0.25 M NaCl, 9% (v/v) 2-propanol, pH 6.9 ± 0.2,
   d) applying the solution of a) to the column obtained in c),
   e) applying a solution containing Calcium-ions at a third concentration to the column obtained in d) comprising 20 mM TRIS-HCl, 0.25 M NaCl, 9% (v/v) 2-propanol, pH 6.9 ± 0.2, and
   f) recovering purified erythropoietin by applying a solution containing Calcium-ions at a fourth concentration to the column obtained in e) comprising 10 mM TRIS-HCl, pH 6.9 ± 0.2,

wherein said first and second Calcium-ion concentrations are the same and said third and fourth Calcium-ion concentrations are the same, lower than said first and second Calcium-ion concentration, wherein said third and fourth Calcium-ion concentration is 0.1 mM or less.

2. Method for producing erythropoietin using a sequence of chromatography steps as follows:

   i) affinity chromatography,
   ii) hydrophobic interaction chromatography,
   iii) chromatography on a stationary phase containing ceramic hydroxyapatite,
   wherein the chromatography on a stationary phase containing hydroxyapatite, is carried out according to claim 1.

3. Method for producing erythropoietin comprising culturing cells containing an EPO-encoding nucleic acid and isolating erythropoietin from the cells or the culture medium, **characterized in that** the isolation of erythropoietin comprises the following steps:

   i) applying the cell supernatant to an affinity chromatography material and recovering/collecting the fractions containing erythropoietin,
   ii) optionally applying the fractions containing erythropoietin from i) to a hydrophobic interaction chromatography material and recovering/collecting the fractions containing erythropoietin,
   iii) applying the fractions containing erythropoietin from i) or ii) to a stationary phase containing ceramic hydroxyapatite and recovering/collecting the fractions containing erythropoietin using a method according to one of claims 1 to 2.

4. Method for producing mono-PEGylated erythropoietin comprising the following steps:

   i) purifying erythropoietin with a method according to one of claims 1 to 2,
   ii) PEGylating erythropoietin using an activated PEG having a molecular weight between 20 kDa and 40 kDa,
   iii) purifying the PEGylated erythropoietin obtained in step ii) with two successive cation exchange chromatography steps using the same stationary phase,
   iv) recovering and thereby producing mono PEGylated erythropoietin from the second stationary phase.

**Patentansprüche**

1. In vitro-Verfahren zur Aufreinigung von Erythropoietin umfassend:

   a) Bereitstellen einer Lösung enthaltend i) Erythropoietin und ii) Calciumionen in einer ersten Konzentration umfassend 20 mM Tris-HCl, 5 mM CaCl$_2$, pH 6,9 $\pm$ 0,2,
   b) Bereitstellen einer Chromatographiesäule enthaltend eine keramisches Hydroxyapatit enthaltende stationäre Phase,
   c) Auftragen einer Calciumionen in einer zweiten Konzentration enthaltenden Lösung auf die Säule aus b) umfassend 20 mM Tris-HCl, 5 mM CaCl$_2$, 0,25 mM NaCl, 9 % (v/v) 2-Propanol, pH 6,9 $\pm$ 0,2,
   d) Auftragen der Lösung aus a) auf die in c) erhaltene Säule,
   e) Auftragen einer Calciumionen in einer dritten Konzentration enthaltenden Lösung umfassend 20 mM Tris-HCl, 0,25 mM NaCl, 9 % (v/v) 2-Propanol, pH 6,9 $\pm$ 0,2, auf die in d) erhaltene Säule, und
   f) Gewinnen von aufgereinigtem Erythropoietin durch Auftragen einer Calciumionen in einer vierten Konzentration enthaltenden Lösung umfassend 10 mM Tris-HCl, pH 6,9 $\pm$ 0,2 auf die in e) erhaltene Säule, wobei jeweils die erste und zweite Calciumionen-Konzentration sowie die dritte und vierte Calciumionen-Konzentration identisch sind und wobei die dritte und vierte Calciumionen-Konzentration niedriger ist als die erste und zweite Calciumionen-Konzentration und wobei die dritte und vierte Calciumionen-Konzentration 0,1 mM oder weniger beträgt.

2. Verfahren zur Herstellung von Erythropoietin unter Verwendung einer Reihe von Chromatographieschritten wie folgt:

   i) Affinitätschromatographie,
   ii) hydrophobe Interaktionschromatographie,
   iii) Chromatographie an einer keramisches Hydroxyapatit enthaltenden stationären Phase,
   wobei die Chromatographie an der Hydroxyapatit enthaltenden stationären Phase nach Anspruch 1 durchgeführt wird.

3. Verfahren zur Herstellung von Erythropoietin umfassend das Züchten von Zellen, die eine EPO codierende Nucleinsäure enthalten, und das Isolieren von Erythropoietin aus den Zellen oder aus dem Kulturmedium, **dadurch gekennzeichnet, dass** das Isolieren des Erythropoietins die folgenden Schritte umfasst:

i) Auftragen des Zellüberstands auf ein Affinitätschromatographiematerial und Gewinnen/Sammeln der Erythropoietin enthaltenden Fraktionen,
ii) gegebenenfalls Auftragen der Erythropoietin enthaltenden Fraktionen aus i) auf ein hydrophobe Interaktionschromatographie-Material und Gewinnen/Sammeln der Erythropoietin enthaltenden Fraktionen,
iii) Auftragen der Erythropoietin enthaltenden Fraktionen aus i) oder ii) auf eine keramisches Hydroxyapatit enthaltende stationäre Phase und Gewinnen/Sammeln der Erythropoietin enthaltenden Fraktionen unter Verwendung eines Verfahrens nach einem der Ansprüche 1 bis 2.

4. Verfahren zur Herstellung von mono-PEGyliertem Erythropoietin umfassend die folgenden Schritte:

i) Aufreinigen von Erythropoietin mittels eines Verfahrens nach einem der Ansprüche 1 bis 2,
ii) PEGylieren von Erythropoietin unter Verwendung von aktiviertem PEG mit einem Molekulargewicht zwischen 20 kDa und 40 kDa,
iii) Aufreinigen des in Schritt ii) erhaltenen PEGylierten Erythropoietins mit zwei aufeinanderfolgenden Kationenaustauschchromatagraphieschritten unter Verwendung der gleichen stationären Phase,
iv) Gewinnen und dabei Produzieren des mono-PEGylierten Erythropoietins von der stationären Phase.

**Revendications**

1. Procédé de purification d'érythropoïétine *in vitro,* comprenant

a) la fourniture d'une solution contenant i) de l'érythropoïétine et ii) des ions calcium à une première concentration comprenant du TRIS-HCl 20 mM, du CaCl$_2$ 5 mM, pH 6,9 $\pm$ 0,2,
b) la fourniture d'une colonne de chromatographie contenant une phase stationnaire contenant une hydroxyapatite céramique,
c) l'application à la colonne de b) d'une solution contenant des ions calcium à une deuxième concentration, comprenant du TRIS-HCl 20 mM, du CaCl$_2$ 5 mM, du NaCl 0,25 M, 9 % (v/v) de 2-propanol, pH 6,9 $\pm$ 0,2,
d) l'application de la solution de a) à la colonne obtenue dans c),
e) l'application à la colonne obtenue dans d) d'une solution contenant des ions calcium à une troisième concentration, comprenant du TRIS-HCl 20 mM, du NaCl 0,25 M, 9 % (v/v) de 2-propanol, pH 6,9 $\pm$ 0,2, et
f) la récupération de l'érythropoïétine purifiée par application à la colonne obtenue dans e) d'une solution contenant des ions calcium à une quatrième concentration, comprenant du TRIS-HCl 20 mM, pH 6,9 $\pm$ 0,2,
où lesdites première et deuxième concentrations d'ions calcium sont identiques et lesdites troisième et quatrième concentrations d'ions calcium sont identiques, inférieures auxdites première et deuxième concentrations d'ions calcium,
lesdites troisième et quatrième concentrations d'ions calcium étant d'au plus 0,1 M.

2. Procédé de production d'érythropoïétine, utilisant une suite d'étapes de chromatographie telle que ci-dessous:

i) une chromatographie par affinité,
ii) une chromatographie par interaction hydrophobe,
iii) une chromatographie sur une phase stationnaire contenant une hydroxyapatite céramique,
la chromatographie sur une phase stationnaire contenant une hydroxyapatite étant effectuée selon la revendication 1.

3. Procédé de production d'érythropoïétine; comprenant la culture de cellules contenant un acide nucléique codant pour l'EPO et l'isolement de l'érythropoïétine à partir des cellules ou du milieu de culture, **caractérisé en ce que** l'isolement de l'érythropoïétine comprend les étapes suivantes:

i) application du surnageant des cellules à un matériau de chromatographie par affinité et récupération/recueil des fractions contenant l'érythropoïétine,

ii) éventuellement application des fractions contenant l'érythropoïétine de i) à un matériau de chromatographie par interaction hydrophobe et récupération/recueil des fractions contenant l'érythropoïétine,

iii) application des fractions contenant l'érythropoïétine de i) ou ii) à une phase stationnaire contenant de l'hydroxyapatite céramique et récupération/recueil des fractions contenant l'érythropoïétine par un procédé selon l'une des revendications 1 à 2.

4. Procédé de production d'érythropoïétine monopégylée, comprenant les étapes suivantes:

i) purification de l'érythropoïétine par un procédé selon l'une des revendications 1 à 2,

ii) pégylation de l'érythropoïétine à l'aide d'un PEG activé ayant une masse molaire entre 20 kDa et 40 kDa,

iii) purification de l'érythropoïétine pégylée obtenue dans l'étape ii) par deux étapes successives de chromatographie par échange de cations utilisant la même phase stationnaire,

iv) récupération et ainsi production d'érythropoïétine monopégylée à partir de la deuxième phase stationnaire.

**Fig. 1**

**Fig. 2**

**Fig. 3**

**Fig. 4**

**Fig. 5**

## Fig. 6

**Fig. 7**

**Fig. 8**

**Fig. 9**

Fig. 10

**Fig. 11**

**Fig. 12**

a)

b)

**Fig. 13**

**Fig. 14**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0148605 A
- EP 0205564 A
- EP 0209539 A
- EP 0411678 A
- EP 0255231 A
- EP 0830376 A
- EP 0267678 A
- EP 0228452 A
- EP 1127063 A
- EP 0358463 A
- EP 1010758 A
- EP 0820468 A
- EP 0984062 A
- EP 0640619 A
- EP 0428267 A

- EP 1037921 A
- EP 1394179 A
- EP 0986644 A
- EP 1428878 A
- EP 1492878 A
- WO 2005121173 A
- WO 9928346 A
- US 2004147431 A
- WO 03080852 A
- EP 0409113 A
- WO 8607494 A
- WO 0044785 A
- WO 9401451 A
- EP 08016678 A

**Non-patent literature cited in the description**

- **HUANG, S.L.** *Proc. Natl. Acad. Sci. USA,* 1984, 2708-2712
- **LAI, P.H. et al.** *J. Biol. Chem.,* 1986, vol. 261, 3116-3121
- **SASAKI, H. et al.** *J. Biol. Chem.,* 1987, vol. 262, 12059-12076
- **MIYAKE, T. et al.** *J. Biol. Chem.,* 1977, vol. 252, 5558-5564
- **NOBUO, I. et al.** *J. Biochem.,* 1990, vol. 107, 352-359
- **LAI, P.H et al.** *J. Biol. Chem.,* 1986, vol. 261, 3116-3121
- **BROUDY, V.C. et al.** *Arch. Biochem. Biophys,* 1988, vol. 265, 329-336
- **ZOU, Z. et al.** *Journal of Chrom.,* 1998, vol. 16, 263-264
- **INOUE, N. et al.** *Biol. Pharm. Bull.,* 1994, vol. 17, 180-184
- **QIAN, R.L. et al.** *Blood,* 1986, vol. 68 (1), 258-262
- **KRYSTAL, G. et al.** *Blood,* 1986, vol. 67 (1), 71-79
- **LANGE, R.D et al.** *Blood Cells,* 1984, vol. 10 (2-3), 305-314
- **SASAKI, R. et al.** *Methods Enzymol.,* 1987, vol. 147, 328-340

- **BEN GHANEM, A. et al.** *Prep. Biochem.,* 1994, vol. 24, 127-142
- **CIFUENTES, A. et al.** *J. Chrom. A,* 1999, vol. 830, 453-463
- **GOKANA, A. et al.** *J. Chromat. A,* 1997, vol. 791, 109-118
- **NIMTZ, M. et al.** *Eur. J. Biochem.,* 1993, vol. 213, 39-56
- Protein Purification Methods, A practical Approach. IRL Press, 1989, 224
- Protein Purification. VCH Publisher, 1989, 207-226
- **NOBUO, I et al.** *J. Biochem.,* 1990, vol. 107, 352-359
- **VERONESE, F.M.** *Biomaterials,* 2001, vol. 22, 405-417
- **FRANCIS, G.E. et al.** *Int. J. Hematol.,* 1998, vol. 68, 1-18
- **DELGADO, C. et al.** *Crit. Rev. Ther. Drug Carrier Systems,* 1992, vol. 9, 249-304
- **LU, Y. et al.** *Reactive Polymers,* 1994, vol. 22, 221-229
- **FELIX, A.M. et al.** poly(ethyleneglycol. ACS Symp. Ser, 1997, vol. 680, 218-238